Europäisches Patentamt

European Patent Office

Office européen des brevets

(11) Veröffentlichungsnummer: **0 326 908**
**A2**

## (12) EUROPÄISCHE PATENTANMELDUNG

(21) Anmeldenummer: 89101163.7

(22) Anmeldetag: 24.01.89

(51) Int. Cl.⁴: **A61M 25/00**

(30) Priorität: 30.01.88 DE 8801101 U

(43) Veröffentlichungstag der Anmeldung:
09.08.89 Patentblatt 89/32

(84) Benannte Vertragsstaaten:
**AT BE CH DE ES FR GB GR IT LI LU NL SE**

(71) Anmelder: **B. Braun Melsungen AG**
**Carl-Braun Strasse**
**D-3508 Melsungen(DE)**

(72) Erfinder: **Schmeller, Nikolaus, Dr. med.**
**Plönniesstrasse 7**
**D-2400 Lübeck(DE)**
Erfinder: **Gerlach, Roland, Dr.-Ing.**
**Kirchweg 9**
**D-3501 Guxhagen(DE)**

(74) Vertreter: **Selting, Günther, Dipl.-Ing. et al**
**Deichmannhaus am Hauptbahnhof**
**D-5000 Köln 1(DE)**

(54) Einführvorrichtung für einen Ureterkatheter.

(57) Die Einführvorrichtung weist eine schlauchförmige Positionierhülse (18) auf, die das rückwärtige Ende des Ureterkatheters (10) abstützt. Durch eine Öffnung (14a) am Katheter (10) ist ein Faden (19) hindurchgefädelt, der an der Positionierhülse (18) mit einer Klemmvorrichtung (21) fixiert ist. Dadurch können über die Positionierhülse (18) Zug und Druck auf den Katheter übertragen werden. Mit der Klemmvorrichtung (21) kann auch der Mandrin (26) fixiert werden. Der Mandrin (26) weist eine steife Seele (29) und einen weicheren Mantel (30) auf. Durch Verschieben der Seele (29) kann die Weichheit der Mandrinspitze verändert werden. Die Einführvorrichtung erlaubt Drehen, Vor- und Zurückziehen des Katheters sowie ein einfaches und schmerzloses Entfernen des Katheters durch Ziehen des Fadens (19).

FIG. 5

## Einführvorrichtung für einen Ureterkatheter

Die Erfindung betrifft eine Einführvorrichtung für einen mit einer nierenseitigen Einrollung versehenen Ureterkatheter nach dem Oberbegriff des Anspruchs 1, bzw. einen Ureterkatheter-Set.

Ureterkatheter sind Drainagekatheter, die zur Schienung in den zwischen Niere und Blase verlaufenden Harnleiter (Ureter) eingeführt werden, um diesen zu schienen und für den Flüssigkeitstransport offen zu halten. Bekannt sind Ureterkatheter, die aus einem flexiblen Schlauch bestehen, der an beiden Enden Einrollungen aufweist, um eine Fixation in den beiden Hohlorganen Niere und Blase zu gewährleisten (DE-GM 79 04 093). Zur Urindrainage sind in den Einrollungen und auf dem geraden Katheterabschnitt Drainagebohrungen angebracht. Zum Einlegen des Katheters in den Körper müssen die Einrollungen gestreckt werden, damit ein Hochführen von der Blase zur Niere ermöglicht wird. Dazu dient ein relativ steifer Mandrin, der in den Katheter eingeführt wird und diesen streckt. Die Plazierung des Katheters mit Mandrin erfolgt über ein Cystoskop. Die Länge des Ureterkatheters wird durch die Länge des gestreckten Katheterabschnitts und die Länge der beiden Einrollungen bestimmt. Die Länge eines Cystoskopes beträgt im allgemeinen 33 cm, so daß der Ureterkatheter vom Cystoskop aufgenommen wird und damit von außen nicht mehr erreichbar ist. Es wird eine zusätzliche Positionierhülse benutzt, die mit ihrem vorderen Ende das rückwärtige Ende des Katheters abstützt und deren rückwärtiges Ende aus dem Cystoskop herausragt. Da ein pathologisch veränderter Harnleiter Engen, Weiten und Krümmungen haben kann, ist in manchen Fällen ein Drehen oder Zurückziehen des Katheters erforderlich. Bei Krümmungen des Harnleiters ist es zweckmäßig, den Rückstelleffekt der Kathetereinrollung zu nutzen, indem der den Katheter streckende Mandrin geringfügig zurückgezogen wird.

Die übliche Verlegetechnik erfolgt so, daß der Katheter, der im Verhältnis zu der sich aus den Einzelabschnitten (Cystoskop, Harnblase, Harnleiter, Nierenbecken) ergebenden instrumentellen und anatomischen Gesamtlänge relativ kurz ist, auf einen langen Mandrin aufgeschoben wird. Der Mandrin wird bis zur Spitze des Katheters vorgeschoben, wodurch der Katheter gestreckt und in gewissem Maße gespannt wird. In diesem Zustand wird der Katheter am blasenseitigen Ende mit einer Klemme fixiert. Dahinter wird die Positionierhülse auf den Mandrin aufgeschoben. Wenn diese Einheit aus Mandrin, Drainagekatheter, Positionierhülse und Klemme in das Cystoskop eingeschoben wird, um den Katheter in die Blase vorzuschieben, wird dieses Vorschieben durch die Fixierklemme behindert. Nach dem Lösen der Fixierklemme wird jedoch die starre Verbindung zwischen Katheter und Mandrin aufgehoben. Das weitere Vorschieben des Katheters in Richtung Niere erfolgt ausschließlich durch den Mandrin. Ein Drehen des Katheters um seine Achse ist nicht mehr möglich. Beim Zurückziehen des Mandrins ist der Katheter bestrebt, sich einzurollen, wodurch ein weiteres Vorschieben behindert oder unmöglich gemacht wird. Die Positionierhülse kann in diesem Zustand ihre Aufgabe, den Katheter gegen den Mandrin abzustützen, nicht mehr wahrnehmen. Zum Entfernen oder zum Wechseln des Katheters ist eine erneute Cystoskopie erforderlich. Der Katheter wird durch das Cystoskop hindurch mit einer Zange gefaßt und herausgezogen. Dies bedeutet für den Patienten neben den unvermeidlichen Schmerzen auch die Gefahr der Traumatisierung der Harnröhre.

Der Erfindung liegt die Aufgabe zugrunde, eine Einführvorrichtung für einen Ureterkatheter zu schaffen, die in jeder Lage des Katheters im Harnleiter auch ein Zurückziehen des Katheters und ein Drehen ermöglicht.

Die Lösung dieser Aufgabe erfolgt erfindungsgemäß mit dem im kennzeichnenden Teil des Anspruchs 1 angegebenen Merkmal.

Bei der erfindungsgemäßen Einführvorrichtung ist der Katheter mit der Positionierhülse während des Einführvorgangs durch einen Faden fest verbunden. Dadurch kann auf die übliche Klemme, die das Cystoskopieren behindert, verzichtet werden. Der Faden kann Zugkräfte von der Positionierhülse auf den Katheter übertragen, so daß durch Ziehen an der Positionierhülse der Katheter auf dem Mandrin gespannt werden kann. Andererseits ist es möglich, durch Drücken gegen die Positionierhülse das nierenseitige Katheterende über das entsprechende Mandrinende hinaus vorzuschieben, damit die dann unverstärkte, gekrümmte Katheterspitze leichter einen Weg durch das Hohlorgan finden kann. Auch Drehungen des Katheters um seine Längsachse herum sind möglich. Durch den dünnen Faden, der den Katheter so gegen die Positionierhülse zieht, daß Katheter und Positionierhülse gewissermaßen einen einzigen Schlauch ohne seitlich abstehende Verdickung bilden, wird die Handhabbarkeit und Führung des Katheters wesentlich erleichtert. Ferner wird auch das Entfernen des Katheters nach Kurzzeitanwendung vereinfacht, weil der Katheter mit dem Faden leicht aus der Harnröhre herausgezogen werden kann.

Der Faden kann durch eine der Drainageöffnungen des Katheters hindurchgefädelt werden, wobei die beiden Fadenstränge durch die Positionierhülse hindurch nach außen führen. Am patientenfernen Ende der Positionierhülse sind die Fadenstränge zurückgebogen und mit einer Klemmvorrichtung an der Positionierhülse fixiert.

Die Klemmvorrichtung ist vorzugsweise so ausgebildet, daß sie die Positionierhülse umgibt und zusammendrückt. Mit einer derartigen Klemmvorrichtung kann nicht nur der Faden in Bezug auf die Positionierhülse fixiert werden, sondern durch Zusammendrückung der Positionierhülse kann auch der Mandrin in dieser festgelegt werden.

Wenn der Mandrin eine steife Seele und einen flexiblen Mantel aufweist und die Seele im Mantel verschiebbar ist, kann die Steifigkeit des vorderen Mandrinendes durch Verschieben der Seele reguliert werden, so daß der Verlegevorgang des Katheters erleichtert wird.

In folgenden wird unter Bezugnahme auf die Zeichnungen ein Ausführungsbeispiel der Erfindung näher erläutert.

Es zeigen:

Fig. 1 eine Seitenansicht der Einführvorrichtung mit Katheter im Lieferzustand,

Fig. 2 eine Seitenansicht einer Rohrspirale zur Aufnahme des Mandrins im Lieferzustand,

Fig. 3 einen Längsschnitt durch den Mandrin,

Fig. 4 eine Klemme zum Fixieren der Fadenenden außerhalb der Harnröhre, und

Fig. 5 das Verlegen des Katheters mit der Katheterspitze im Nierenbecken unter Verwendung der Einführvorrichtung.

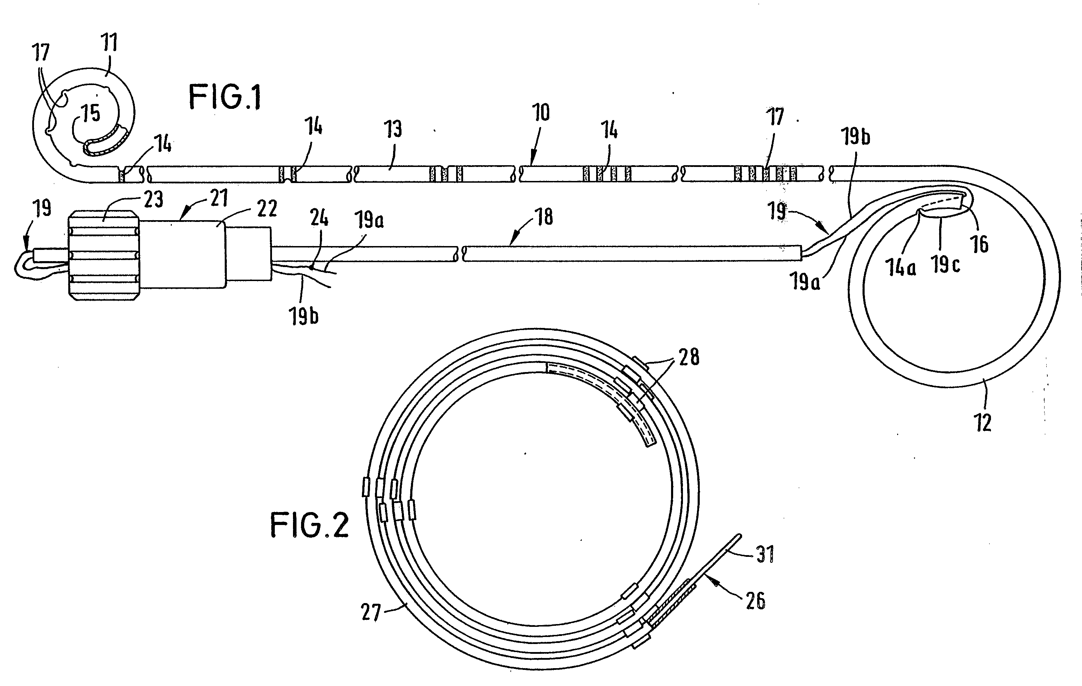

Gemäß Fig. 1 ist ein rohrförmiger Katheter 10 aus einem flexiblen Kunststoffschlauch vorgesehen, der an einem Ende eine nierenseitige Einrollung 11 und am anderen Ende eine blasenseitige Einrollung 12 aufweist. Die Einrollungen 11,12 sind nach entgegengesetzten Seiten gerichtet. Durch Einführen eines Mandrins in den Katheter 10 können die Einrollungen gestreckt werden. Wird der Mandrin zurückgezogen, dann bilden sich die Einrollungen 11,12 zurück. Zwischen den Einrollungen 11,12 befindet sich der geradlinige Mittelabschnitt 13. Über die Katheterlänge sind Markierungen 14 angebracht, um die Lage des Katheters kontrollieren zu können. Am vorderen (nierenseitigen) Ende ist die Katheterspitze 15 geschlossen, während das rückwärtige (blasenseitige) Katheterende 16 offen ist.

Am Katheter sind Drainageöffnungen 17 vorgesehen. Der insoweit beschriebene Katheter 10 ist bekannt.

Zum Verlegen des Katheters dient die Positionierhülse 18, die aus einem Schlauch besteht, dessen Außendurchmesser etwa gleich demjenigen des Katheters 10 ist. Die Positionierhülse 18 ist flexibel, jedoch etwas steifer als der Katheter 10 und insbesondere knickfester. Durch die Positionierhülse 18 erstrecken sich die beiden Stränge eines Fadens 19, der an dem dem Katheter 10 zugewandten Ende eine Schleife 19c bildet, so daß die Enden der Fadenstränge aus dem dem Katheter abgewandten Ende der Positionierhülse 18 herausragen. Der eine Fadenstrang 19a führt in das offene Ende 16 des Katheters hinein und aus einer seitlichen Öffnung 14a in der Nähe des offenen Endes 16 heraus. Der andere Fadenstrang 19b liegt also ein kurzes Stück seitlich am Katheter 10 an.

Auf dem rückwärtigen Ende der Positionierhülse sitzt eine Klemmvorrichtung 21, die die Positionierhülse 18 umgibt und diese radial zusammendrücken kann. Die Klemmvorrichtung 21 weist ein Gehäuse 22 und eine Mutter 23 auf. Wenn die Mutter 23 relativ zu dem Gehäuse 22 gedreht wird, erfolgt durch ein (nicht dargestelltes) Klemmelement das radiale Zusammendrücken der Positionierhülse 18. Die beiden Fadenstränge 19a,19b, die aus dem patientenfernen Ende der Positionierhülse 18 herausragen, sind um 180° umgelenkt und außerhalb der Positionierhülse durch die Klemmvorrichtung 21 geführt, so daß sie beim Festspannen der Klemmvorrichtung an der Außenseite der Positionierhülse fixiert werden. Die Enden der Fadenstränge 19a und 19b ragen aus dem patientenseitigen Ende der Klemmvorrichtung 21 heraus. An dem Fadenstrang 19a befindet sich eine Verdickung 24 in Form eines Knotens u.dgl. oder eine andere Markierung, durch die derjenige Fadenstrang 19a bezeichnet ist, der in das offene Ende 16 des Katheters 10 hereinführt. An diesem Fadenstrang 19a muß gezogen werden, wenn der Faden vom Katheter 10 gelöst werden soll. Würde an dem anderen Fadenstrang 19b gezogen werden, würde die Gefahr der Verklemmung des Fadens bestehen.

In Lieferzustand der Vorrichtung ist der Faden 19 so gespannt, daß das patientenferne Ende 16 des Katheters 10 gegen das patientenseitige Ende der Positionierhülse 18 stößt, d.h. die Enden der Fadenstränge sind möglichst weit durch die Klemmvorrichtung 21 hindurchgezogen. In diesem Zustand kann der Mandrin 26 durch die Positionierhülse 18 und den Katheter 10 hindurchgeschoben werden, bis sein vorderes Ende gegen die Katheterspitze 15 stößt, während sein rückwärtiges Ende noch aus dem patientenfernen Ende der Positionierhülse 18 herausragt.

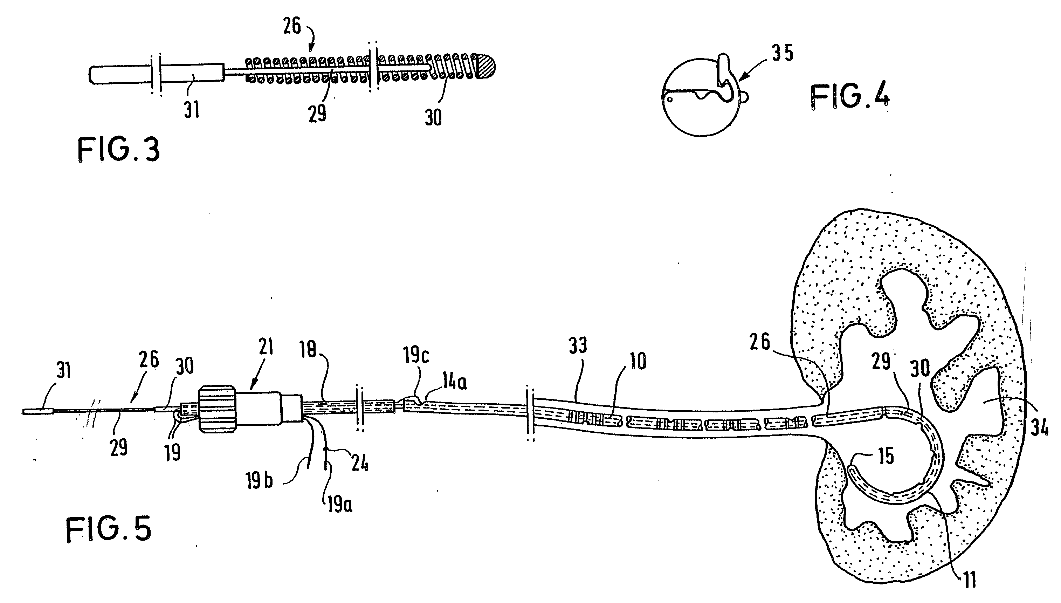

Der Mandrin 26 befindet sich in einer als Aufbewahrungsgehäuse dienenden Rohrspirale 27 (Fig. 2), deren Windungen durch Klemmen 28 in spiralförmiger Konfiguration zusammengehalten sind. Der Mandrin 26 weist eine aus monofilem Draht bestehende Seele 29 (Fig. 3) auf, die relativ steif ist und von einem relativ flexiblen Mantel 30 aus schraubenförmig gewickeltem Draht umgeben ist. Das rückwärtige Ende der Seele 29 ragt aus dem Mantel 30 heraus und trägt ein starres Griffstück 31, um die Seele 29 in dem Mantel 30 verschieben zu können. Durch Verschiebung der Seele 29 kann die Länge des vorderen weichen Endes des Mandrins verändert werden.

Zum Verlegen des Katheters wird der Mandrin 26 aus der Rohrspirale 27 herausgezogen und anschließend mit seinem vorderen Ende in die Positionierhülse 18 und den Katheter 10 eingeführt, wobei das rückwärtige Ende des Mandrins mit dem Griffstück 31 aus der Positionierhülse herausragt. Der Mandrin 26 wird bis zur Katheterspitze 15 vorgeschoben und anschließend durch Festziehen der Klemmvorrichtung 21 in der Positionierhülse 18 fixiert, wobei der Faden 19 in gespanntem Zustand mitfixiert wird. Der Katheter 10 wird durch ein (nicht dargestelltes) Cystoskop hindurch im Harnleiter 33 vorgeschoben bis das nierenseitige Katheterende das Nierenbecken 34 erreicht. Während des Vorschiebens kann die Seele 29 im Mandrinmantel 30 in eine Stellung gebracht werden, die ein teilweises Krümmen der Einrollung 11 des Katheters zuläßt.

Nach Verlegung des Katheters 10 wird die Klemmvorrichtung 21 gelöst und der Mandrin 26 aus dem Katheter 10 und der Positionierhülse 18 herausgezogen. Im Nierenbecken bildet sich dann die Einrollung 11 gemäß Fig. 5 vollständig aus. Soll der Katheter nur für Kurzzeitanwendung benutzt werden, kann die Positionierhülse 18 entfernt werden, während der Faden 19 im Patienten verbleibt. Der Faden wird durch die Harnröhre nach außen geführt und mit der in Fig. 4 dargestellten Klemme 35 fixiert, die beide Fadenstränge 19a und 19b festhält. Durch Ziehen an dem Fadenstrang 19a ist jederzeit ein Entfernen des Katheters 10 möglich.

Bei Langzeitanwendung des Katheters wird der Faden 19 vor dem Herausziehen der Positionierhülse 18 entfernt, um eine Infektion durch Bakterien entlang des Fadens zu verhindern.

## Ansprüche

1. Einführvorrichtung für einen mit einer nierenseitigen Einrollung (11) versehen Ureterkatheter, mit einer den Katheter (10) endseitig abstützenden Positionierhülse (18) und einem in Positionierhülse (18) und Katheter (10) einschiebbaren Mandrin (26), **dadurch gekennzeichnet,** daß der Katheter (10) mit der Positionierhülse (18) über einen durch die Positionierhülse (18) hindurch verlaufenden Faden (19) verbunden ist.

2. Einführvorrichtung nach Anspruch 1, dadurch gekennzeichnet, daß der Faden (19) durch eine Öffnung (14a) des Katheters (10) gefädelt ist und doppelt durch die Positionierhülse (18) hindurch verläuft und daß am patientenfernen Ende der Positionierhülse (18) eine Klemmvorrichtung (21) zum Fixieren der beiden Fadenstränge (19a,19b) vorgesehen ist.

3. Einführvorrichtung nach Anspruch 2, dadurch gekennzeichnet, daß die Klemmvorrichtung (21) die Positionierhülse (18) umgibt und zusammendrückt.

4. Einführvorrichtung nach Anspruch 2 oder 3, dadurch gekennzeichnet, daß an einem der aus der Klemmvorrichtung (21) herausragenden Fadenenden eine Verdickung (24) vorgesehen ist.

5. Einführvorrichtung nach einem der Ansprüche 1 bis 4, dadurch gekennzeichnet, daß der Mandrin (26) eine relativ steife Seele (29) und einen relativ flexiblen Mantel (30) aufweist, wobei die Seele (29) in dem Mantel (30) verschiebbar ist.

6. Einführvorrichtung nach Anspruch 5, dadurch gekennzeichnet, daß die Seele (29) an dem aus dem Mantel (30) herausragenden patientenfernen Ende ein starres Griffstück (21) aufweist.

7. Einführvorrichtung nach einem der Ansprüche 1 bis 6, dadurch gekennzeichnet, daß eine Klemme (35) zum Fixieren der Fadenenden außerhalb der Harnröhre vorgesehen ist.

FIG.1

FIG.2

EP 0 326 908 A2

FIG.3

FIG.4

FIG.5

EP 0 326 908 A2